# EUROPÄISCHE PATENTANMELDUNG

(11) **EP 0 691 402 A2**
(43) Veröffentlichungstag der Anmeldung: **10.01.1996**
(21) Anmeldenummer: 95108178.5
(22) Anmeldetag: 29.05.1995
(51) Int. Cl.: C12N 15/11, C12N 9/00, C12N 15/63, C12N 15/83, C12N 15/90

(54) **Modifizierte Satelliten-RNAs und Satelliten-Viren als Träger adaptierter Ribozyme**

(30) Priorität: 10.06.1994 DE 4420298
(71) Anmelder: Hoechst Schering AgrEvo GmbH, D-13509 Berlin (DE)
(72) Erfinder: Loss, Peter Dr., D-65527 Niedernhausen (DE); Schneider , Dr. Rudolf, D-65527 Niedernhausen, (DE)

(57) **Zusammenfassung**

Die vorliegende Erfindung betrifft enzymatische RNA-Moleküle, die mit Satelliten-Viren oder Satelliten-RNA verknüpft sind, Verfahren zu ihrer Herstellung, die entsprechenden kodierenden DNA-Fragmente sowie mit dieser DNA transformierten Zellen. Die Erfindung betrifft außerdem die Verwendung der erfindungsgemäßen Ribozyme als antivirale Mittel.

SEQUENZPROTOKOLL

(1) ALLGEMEINE INFORMATION:

(i) ANMELDER:
(A) NAME: Hoechst Schering AgrEvo GmbH
(B) STRASSE: -
(C) ORT: Frankfurt
(D) BUNDESLAND: -
(E) LAND: Deutschland
(F) POSTLEITZAHL: 65926
(G) TELEPHON: 069-305-5596
(H) TELEFAX: 069-357175
(I) TELEX: 4 1234 700 ho d

(ii) ANMELDETITEL: Modifizierte Satelliten-RNAs und
Satelliten-Viren als Träger adaptierter Ribozyme

(iii) ANZAHL DER SEQUENZEN: 2

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 336 Basenpaare
(B) ART: Nukleinsäure
(C) STRANGFORM: Einzel
(D) TOPOLOGIE: linear

(ii) ART DES MOLEKÜLS: RNS (genomisch)

(vi) URSPRÜNLICHE HERKUNFT:
(A) ORGANISMUS: Virus

(ix) MERKMALE:
(A) NAME/SCHLÜSSEL: exon
(B) LAGE: 1..336

(xi) SEQUENZBESCHREIBUNG: SEQ ID NO: 1:

GUUUUGUUUG AUGGAGAAUU GCGUAGAGGG GUUAUAUCUA CGUGAGGAUC CGUCACUCGG 60

CGGUGUGGGU UACCUCCCUG CUACGGCGGG UUGAGUUGAC GCACCUCGGA CUCGGGGACC 120

GCUUGGUUUG CGAGUAUCGU CCGCAUUCUU AGCACUACGC GCCAAUUUGA GCCCCCGCCU 180

AGUUUGCUAG CAGCACACGC UCAUGGUUUG CCGUUACCAU GGAAUUUCGA AAGAAACACU 240

CUGUUAGGUG GUAUCGUGGA UGACGCACGC AGGGAGAGGC UUAGACUUAG GUUAUGCUGA 300

UCUCCGUGAA UGUCUACACA UUCCUCUACA GGACCC 336

(2) INFORMATION ZU SEQ ID NO: 2:

(i) SEQUENZ CHARAKTERISTIKA:
(A) LÄNGE: 383 Basenpaare
(B) ART: Nukleinsäure
(C) STRANGFORM: Einzel
(D) TOPOLOGIE: linear

(ii) ART DES MOLEKÜLS: RNS (genomisch)

(vi) URSPRÜNLICHE HERKUNFT:
(A) ORGANISMUS: Virus

(ix) MERKMALE:
(A) NAME/SCHLÜSSEL: exon
(B) LAGE: 1..383

(xi) SEQUENZBESCHREIBUNG: SEQ ID NO: 2:

GUUUUGUUUG AUGGAGAAUU GCGUAGAGGG GUUAUAUCUA CGUGAGGAUC CGUCACUCGG 60

CGGUGUGGGU UACCUCCCUG CUACGGCGGG UUGAGUUGAC GCACCUCGGA CUCGGGGACC 120

GCUUGGUUUG CGAGUAUCGU CCGNNNNNNN NNNNNNNNNN NNNNNNNNNN NNNNNNNNNN 180

NNNNNNNNNN CAUUCUUAGC ACUACGCGCC AAUUUGAGCC CCCGCCUAGU UUGCUAGCAG 240

CACACGCUCA UGGUUUGCCG UUACCAUGGA AUUUCGAAAG AAACACUCUG UUAGGUGGUA 300

UCGUGGAUGA CGCACGCAGG GAGAGGCUUA GACUUAGGUU AUGCUGAUCU CCGUGAAUGU 360

CUACACAUUC CUCUACAGGA CCC 383

## Beschreibung

### 1. Einleitung

Die vorliegende Erfindung betrifft enzymatische RNA-Moleküle, die mit Satelliten-Viren oder Satelliten-RNA verknüpft sind, Verfahren zu ihrer Herstellung, die entsprechenden kodierenden DNA-Fragmente sowie mit dieser DNA transformierten Zellen. Die Erfindung betrifft außerdem die Verwendung der erfindungsgemäßen Ribozyme als antivirale Mittel.

Der Begriff "Ribozyme" wurde zur Beschreibung von RNA-Sequenzen mit enzymatischer Aktivität geprägt, die RNA-Sequenzen intra- oder intermolekular spalten können (Symons, 1991; Edgington, 1992). Die eigentliche Spaltung besteht aus zwei Schritten: zuerst interagieren die komplementären Sequenzbereiche des Ribozyms mit der Substrat-RNA, dann wird die Substrat-Sequenz durch die nukleolytische Aktivität einer mehr oder weniger konservierten sogenannten katalytischen Region gespaltet.

Für die Effektivität der eingesetzten Ribozyme spielt die Interaktion zwischen Ribozym und Substrat eine wesentliche Rolle. Diese wird durch die intramolekularen Wechselwirkungen der Ribozym-Moleküle und der Substrat-Moleküle unter- und miteinander beeinflußt. Eine weitere wesentliche Rolle spielt die Kompartimentierung eukaryotischer Zellen. Ribozym und zu hemmendes Substrat müssen im gleichen Kompartiment vorhanden sein, damit eine Interaktion überhaupt stattfinden kann.

Aufgrund ihrer Eigenschaften bieten sich Ribozyme zur selektive Zerstörung viraler RNA im Innern von Zellen an. So benutzen die überwiegendende Mehrheit der pflanzenpathogenen Viren RNA als Informationsüberträgerin. Diese RNA ist meist einsträngig, es kommt jedoch auch doppelsträngige RNA vor. Die Vermehrung der Viren erfolgt in der Wirtszelle. Da Viren keine selbständigen Organismen sind, bedienen sie sich lebender Zellen und sind bei der Replikation der Nukleinsäure und der Synthese der Proteinhülle auf diese Wirtszelle angewiesen.

Es gibt jedoch auch virale Sequenzen bzw. Viren, deren Fortpflanznung nicht nur von der Wirtszelle, sondern zusätzlich von der Anwesenheit eines weiteren Virus abhängig ist.

So sind Satelliten-RNAs kleine RNA-Moleküle, die nicht für eigene Proteine kodieren und nicht durch wirtseigene Proteine repliziert werden (Roossinck et al., 1992). Ihre Replikation bedarf vielmehr der Gegenwart spezieller sog. Helferviren, die zum einen die geeigneten Proteine der Replikation (RNA-abhängige RNA-Polymerase; Helikase) zur Verfügung stellen, zum anderen auch für die Hüllproteine kodieren, die für die Verpackung und somit die systemische wie auch außerindividuelle Verbreitung dieser Moleküle notwendig sind.

Im Gegensatz zu den Satelliten-RNAs kodieren Satelliten-Viren zwar ebenfalls nicht für die zur Replikation erforderlichen Proteine, wohl aber für ein Hüllprotein (Kaper and Collmer, 1988). Satelliten-RNAs und Satelliten-Viren wurden bisher in bedeutender Zahl in der Assoziation mit Pflanzenpathogenen gefunden (Roosinck et al., 1992;), jedoch konnten neben diesen mittlerweile auch human- und tierpathogene Satelliten-Viren wie das "Adeno-Associated Virus (AAV)" (Berns, 1990), ein DNA-Satelliten-Virus, und das "Hepatitis Delta Virus (HDV)" (Taylor, 1990), ein RNA-Satelliten-Virus, nachgewiesen werden.

Von pflanzlichen Satelliten-RNAs ist bekannt, daß sie nicht nur die Anwesenheit eines sogenannten Helfer-Virus benötigen, sondern daß sie auch in der Lage sind, die Pathogenität des Helfer-Virus mehr oder weniger stark zu modulieren (Kaper et al., 1988; Collmer and Howell, 1992).

Diese Modulation kann einerseits zu einer vollständigen Abschwächung der Symptome, aber auch zu einer dramatischen Verstärkung der Symptome führen, die durch die normale alleinige Virusinfektion eingetreten wären. Die Art und Stärke der Modulation ist von der Kombination der drei beteiligten Komponenten - Helfer-Virus, Satelliten-RNA und Wirtspflanze - beeinflußt. Aus diesem Grunde werden schon seit einiger Zeit Versuche mit dem Ziel durchgeführt, eine Repression viraler Symptome durch eine zusätzliche Infektion mit derartigen nachgewiesenermaßen symptomabschwächend wirkenden Satelliten-RNAs zu erzielen (Tien et al., 1987; Montasser et al., 1991).

Bisher wurden symptomabschwächend und symptomverstärkend wirkende Satelliten-Viren beschrieben, wobei die unterschiedlichen Effekte auf die Symptommodulation des jeweiligen Helfer-Virus lediglich in dem Austausch einiger Nukleotide begründet ist, und die Mechanistik der Symptommodulation zum gegenwärtigen Zeitpunkt noch nicht genau verstanden sind (Palukatitis, 1988; Kaper et al., 1990; Sleat and Palukaitis, 1990; Roossinck et al., 1992). Die verschiedenen Satelliten-RNAs eines Helfer-Virus unterscheiden sich nicht nur hinsichtlich ihrer Sequenz sondern auch bezüglich ihrer Größe voneinander, wenn sich auch die Größenvarianz nur innerhalb eines engen Spektrums abspielt.

### 2. Beschreibung der Erfindung

Überraschenderweise konnte gezeigt werden, daß sowohl das Problem der Lokalisierung in das richtige Kompartiment als auch die Steuerung der Aktivität durch die Insertion ausgewählter Ribozym-Sequenzen in eine bestimmte Sequenzumgebung gelöst werden kann. Diese Sequenzumgebung ist dadurch gekennzeichnet, daß sie natürlicherweise im gleichen Kompartiment repliziert wie das Virus, das durch sie zerstört werden soll.

Die vorliegende Erfindung betrifft modifizierte Ribozyme, enthaltend
(a) nukleolytisch aktive RNA-Sequenzen, die virale genomische RNA, mRNA oder hnRNA spalten und
(b) Satelliten-Viren oder Satelliten-RNA, die mit den nukleolytisch aktiven RNA-Sequenzen funktionell verknüpft sind.

Die vorliegende Erfindung betrifft ein Verfahren zur Herstellung modifizierter Ribozyme, dadurch gekennzeichnet, daß
(a) nukleolytisch aktive RNA-Sequenzen, die virale genomische RNA, mRNA oder hnRNA spalten können
(b) mit Satelliten-Viren oder Satelliten-RNA funktionell verknüpft werden.

Die Erfindung betrifft insbesondere Verfahren, in denen Ribozym-Sequenzen in Satelliten-RNAs sowie in die RNA von Satelliten-Viren pflanzenpathogener, tierpathogener und humanpathogener Viren integriert werden.

Als Ausgangbasis zur Herstellung dieser modifizierten Ribozyme können die bisher bekannten Gruppen von Ribozymen, wie z.B. die Hammerhead-oder Hairpin-Motive aufweisenden Ribozyme, die Ribozyme vom Typ der Gruppe-I-Introns, RNAseP-ähnliche Sequenzen oder Hepatitis-Delta-Virus-Sequenzmotive verwendet werden.

Die Erfindung betrifft ferner die für diese modifizierten Ribozyme kodierenden DNA-Fragmente.

Die Ribozymsequenzen sind im Falle der Ausschaltung von RNA-Viren vorzugsweise gegen deren genomische RNA, insbesondere bevorzugt gegen die als Replikations-intermediat in der Wirtszelle zu dieser in der komplementären Sequenz vorliegende RNA gerichtet.

Die erfindungsgemäßen Ribozyme können als antivirale Arzneimittel gegen pflanzliche, animale oder humane Viren verwendet werden.

Die Erfindung betrifft insbesondere Verfahren zur Behandlung von Pflanzen, dadurch gekennzeichnet, daß
(a) Pflanzenzellen mit der für die enzymatischen RNA-Moleküle kodierenden DNA transformiert werden,
(b) die DNA stabil in das Genom der Pflanzen integiert wird und
(c) aus diesen Zellen Pflanzen regeneriert werden.

Die Erfindung betrifft außerdem Verfahren zur Behandlung von Pflanzen, dadurch gekennzeichnet, daß
(a) Pflanzen mit den modifizierten Ribozymen behandelt werden.

Der Begriff Behandlung bedeutet in diesem Zusammenhang, daß die modifizierten Ribozyme nicht in der Zelle gebildet werden, sondern auf die Pflanzen aufgetragen werden. Die Pflanzen können aber auch wie in 2.3 ausgeführt sowohl transgen in bezug auf ein Ribozym sein und zusätzlich mit RNA-Sequenzen behandelt werden.

Die Erfindung betrifft ferner die Verwendung der erfindungsgemäßen Ribozyme oder Teilen davon zur Behandlung von Pflanzen gegen Viren.

Der entscheidende Vorteil gegenüber der Anwendung konventioneller Ansätze der Ribozymtechnik besteht in folgenden Punkten:
1. Die Ribozyme liegen im gleichen Kompartiment vor wie das Virus, das durch sie zerstört werden soll, da die Satelliten-RNAs und Satelliten-Viren durch die Replikasen und die Helikasen der korrespondierenden Helfer-Viren repliziert werden, wobei die Replikation dieser Viren im Cytoplasma der Wirtszelle stattfindet.
2. Die Vermehrung der Satelliten-RNA bzw. der RNA der Satelliten-Viren erfolgt solange, wie auch die virale Replikase vorliegt, und somit die virale RNA repliziert wird.
3. Die Satelliten-RNA bzw. die RNA der Satelliten-Viren wird bei einer erfolgenden Neuinfektion eines weiteren Wirts-Individuums mit dem Virus mitübertragen und ist somit in diesem dann ebenfalls aktiv.
4. Bei dem Einsatz der Ribozyme unter Verwendung von Satelliten-RNAs bzw. Satelliten-Viren kann der transgene prophylaktische individuelle Schutz umgangen werden, da eine Einbringung dieser Moleküle auch nach einer Infektion noch möglich ist. Dies kann in Form von in vitro-Transkripten bzw. in Form von "reassemblierten" Virionen erfolgen.
5. Von RNA-Viren ist bekannt, daß sie einer relativ hohen Mutationsrate unterliegen. Durch den erst nach einer Infektion erfolgenden Einsatz eröffnet sich durch vorangehende Sequenzbestimmung (mit Hilfe der RT-PCR und durch anschließende direkte Sequenzierung der PCR-Produkte) die Möglichkeit einer Anpassung der integrierten Ribozymsequenz an die aktuelle Sequenz der viralen RNAs. Somit ist man durch diesen Ansatz in der Lage der genetischen Drift der viralen RNA entgegenzuwirken. Neben dem Einsatz nur einer aktualisierten Ribozym-Sequenz besteht auch die Möglichkeit des Einsatzes mehrerer verschiedener Sequenzen in einem Organismus.
6. Der Einsatz von riboyzmenthaltenden Satelliten-RNAs oder Satelliten-Viren ist nicht auf die Zerstörung korrespondierender Helfer-Viren limitiert. Vielmehr kann ein transgener Organismus, der die Nukleinsäure zur Synthese der notwendigen Proteine (des Helfer-Virus) als chromosomalen Bestandteil enthält in der oben beschriebenen Weise (Punkt 4 Satz 2) mit der modifizierten Satelliten-RNA bzw. der modifizierten RNA eines Satelliten-Virus infiziert werden. Dies führt dann zur Spaltung spezifischer endogen kodierter RNA-Sequenzen im Cytoplasma der Wirtszellen. Hierdurch lassen sich entwicklungsspezifische Prozesse wie z.B. die Reifungsprozesse bei früchtebildenden Nutzpflanzen ab einem beliebigen optimalen Zeitpunkt beeinflussen. Durch diese Anwendung kann das Kompartimentierungsproblem der beiden wechselwirkenden Partner (der ribozymkodierenden RNA und der zu spaltenden Substrat-RNA) umgangen werden. Essentiell ist hierbei lediglich, daß die für die Virus-Proteine kodierenden im Wirts-Genom integrierten viralen Genomsequenzen nicht vollständig sind, das heißt, der synthetisierten viralen RNA fehlen die zur eigenständigen Replikation und zur Verpackung erforderlichen Sequenzen.

Die Transformation der Pflanzen kann nach den bekannten Methoden durchgeführt werden, wie sie z.B. in Maniatis et al. (Molecular Cloning: A Laboratory Manual, 1982, New York) beschrieben werden. Diese Methoden umfassen die Transformation pflanzlicher Zellen mit T-DNA mit Hilfe der Agrobakteriumtechnologie (EP 116 718), direkte Protoplastentransformation (EP 164 575), Elektroporation oder ballistische Methoden.

Die erfindungsgemäßen DNA-Sequenzen können durch Insertionen, Deletionen und Substitutionen verändert und modifiziert werden, wenn dadurch keine für das Ribozym selbst relevanten Sequenzen verändert werden. Geeignete Expressionsvektoren sind in der Literatur beschrieben. Sinnvollerweise sollten die Vektoren ein Selektionsmarkergen enthalten. Weder die Wahl der Transformationsmethode noch die Wahl der Vektoren hat einen entscheidenden Einfluß auf die Erfindung.

Sowohl im Fall der Satelliten-RNAs wie auch im Fall der Satelliten-Viren ist eine Einbringung von Fremd-RNA in die nichtkodogenen RNA-Bereiche möglich. Dies kann im Falle der Satelliten-RNAs über die RNA verteilt erfolgen, und die jeweilige Insertion muß im einzelnen Fall auf eventuelle negative Wechselwirkungen mit der Replikation und der Stabilität dieser RNA überprüft werden. Im Fall der Satelliten-Viren kann der nichtkodogene Bereich der RNA zur möglichen Einklonierung fremder Nukleinsäuresequenzen verwendet werden.

Bei den bekannten pflanzlichen Satelliten-Viren befinden sich die kodogenen Sequenzen für das Capsid-Protein am 5'-Ende der RNA, so daß eine Einklonierung zusätzlicher RNA vorzugsweise im weiter zum 3'-Ende hin gelegenen Bereich durchgeführt wird.

Die erfindungsgemäßen Ribozymen können vorteilhaft für die folgenden Aufgaben eingesetzt werden: Die Spaltung elementarer viraler RNA-Sequenzen mit dem Ziel der vollständigen Inhibition der viralen Replikation und die Spaltung endogen kodierter wirtseigener RNA-Transkripte.

### 2.1 Verwendung von Satelliten-RNAs zur Wachstumsinhibitions von Viren

Die erfindungsgemäßen Ribozyme werden vorzugsweise mit den in Tabelle 1 aufgelisteten pflanzlichen Satelliten-RNAs hergestellt, in dem man die Ribozymkodierenden Sequenzen mit den für die Satelliten-RNAs kodierenden DNA-Sequenzen verknüpft.

Angegeben sind hierbei die Helferviren (in eckigen Klammern die Virusfamilie), die nachgewiesenermaßen über Satelliten-RNAs verfügen, sowie die Größen (Anzahl der Nukleotide) der korrespondierenden Satelliten-RNAs.

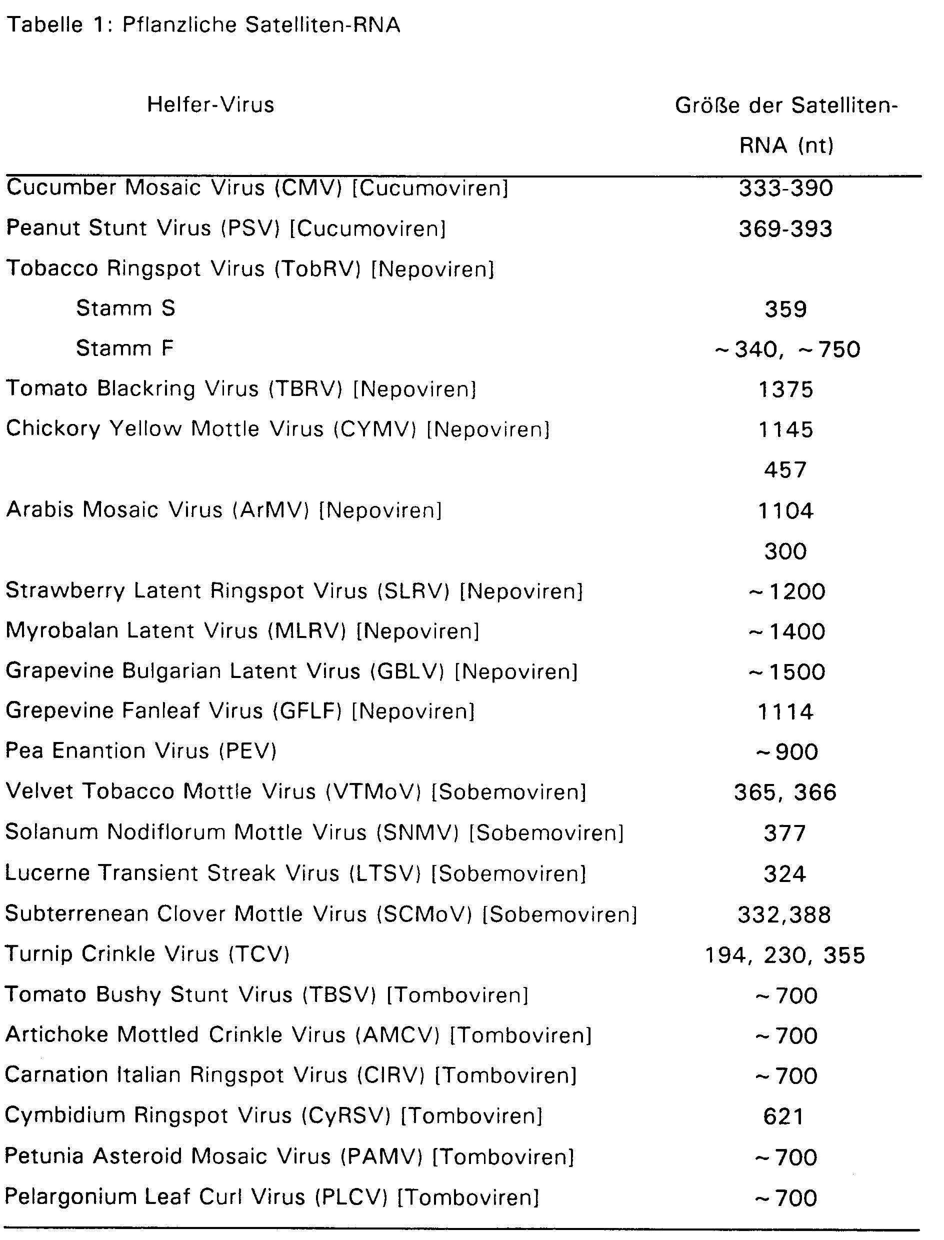

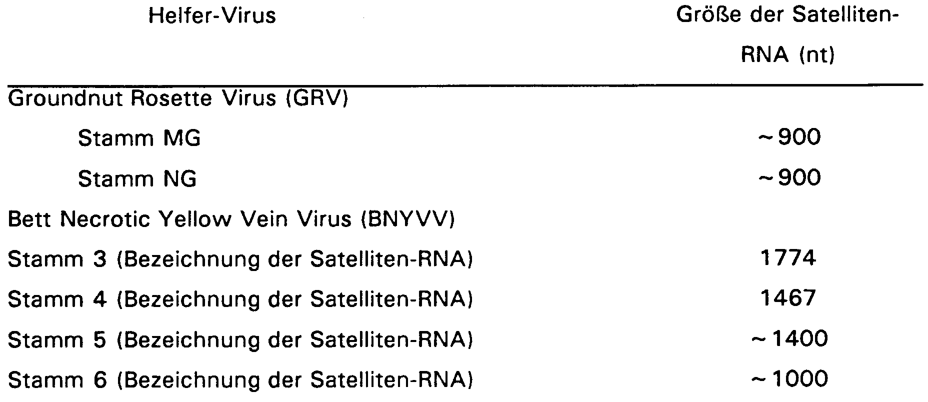

Für die Satelliten-RNA des Cucumber Mosaic Virus (CMV), die in der Literatur als CARNA 5 bezeichnet wird, sind Größen von 333 bis 390 Nukleotide beschrieben (Roossinck et al., 1992). Durch den Vergleich aller bisher beschriebenen CARNAS Satelliten-RNAs wird jedoch deutlich, daß bei allen Subspezies zwischen stark konservierten, bedingt variablen und stark variablen Sequenzbereichen unterschieden werden kann.

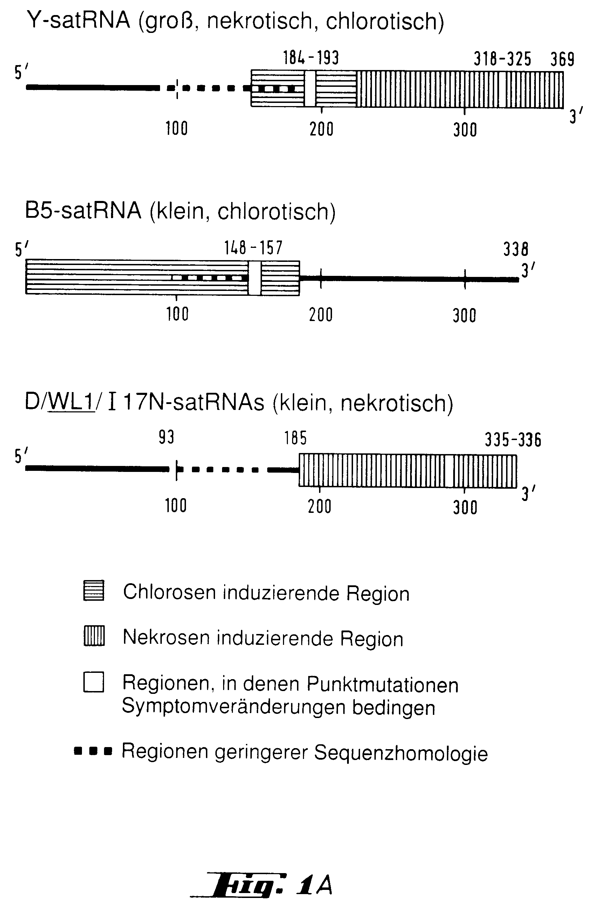

In Abb. 1 sind vergleichend verschiedene Satelliten-RNA-Subspezies aufgeführt, wobei die symptombestimmenden Bereiche extra hervorgehoben sind (Abb. 1 A). Gleichermaßen ist der hochvariable Sequenzbereich der Satelliten-RNAs gestrichelt dargestellt. In Abb. 1B ist die Klonierung der Ribozym-Sequenzen in derlei Satelliten-RNAs am Beispiel des CARNA5 schematisch dargestellt. In eine einzelne Schnittstelle (BsmI), die auf dem gewählten Vektor nicht vorhanden ist, kann in die cDNA der Satelliten-RNA/WL 1 eine beliebige Sequenz eingeführt werden, die eine Spaltungsaktivität gegen einen Sequenzbereich des Helfer-Virus aufweist.

Deutlich ist erkennbar, daß die resultierende RNA des CARNA5/WL1/RIB zwar etwas länger ist als die RNA der CARNA5/Y-RNA, jedoch immer noch kleiner als die mit 390 Nukleotiden beschriebenen längsten CARNA5-RNAs. Die CARNA5-cDNA wurde vor der Ribozym-Klonierung in einen Transkriptionsvektor insertiert, der keine BsmI-Schnittstelle aufweist (pBLUESCRIPT SK).

Die Einklonierung der cDNA in den Transkriptionsvektor erfolgt gerichtet über die XhoI/SmaI-Schnittstellen, wobei eine in vitro-Transkription nach einer SmaI-Linearisierung erfolgen kann. Derlei in vitro-Transkripte sind infektiös und können mit Hilfe beschriebener Standardmethoden in die Wirtspflanze eingebracht werden. Wichtig ist in diesem Zusammenhang, daß das 3'-Ende der resultierenden Transkripte keine zusätzlichen Nukleotide enthalten darf wohingegen am 5'-Ende der resultierenden Transkripte zusätzliche Nukleotide vorliegen dürfen (Kurath und Palukaitis, 1987).

In den Tabellen 1 und 2 ist die Klonierung eines exemplarischen Ribozyms gegen die RNA2 des CMV-Fny-Stammes in die cDNA des CARNA5/WL1 sequenzgenau dargestellt. Hierbei wird zuerst die Sequenz des CABNA5/WL1-Wildtyps gezeigt, in der die Klonierungsstelle für die Insertion beliebiger Ribozym-Sequenzen durch Unterstreichung hervorgehoben wurde.

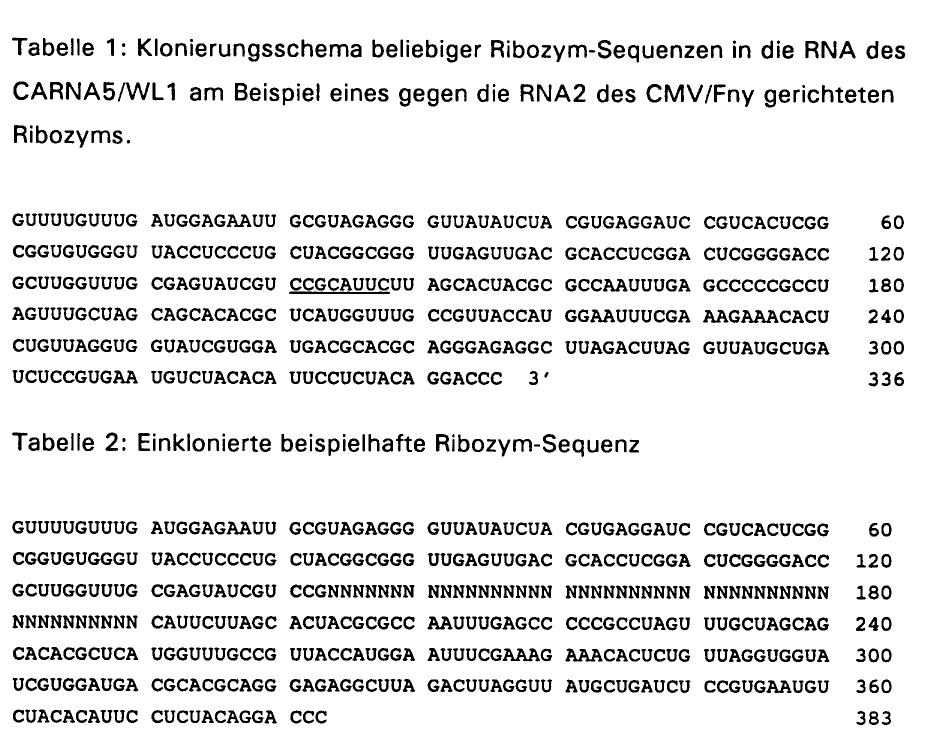

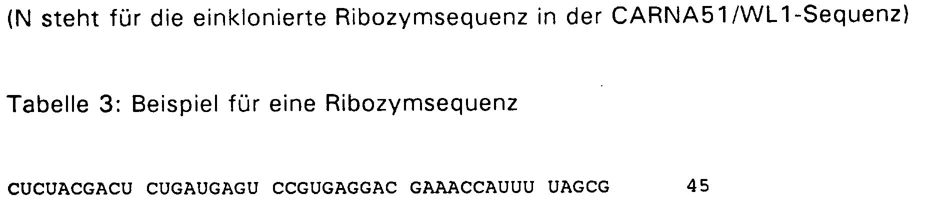

Die bei der in vitro-Transkription entstehenden zusätzlichen 5'-seitigen Nukleotide sind in dieser Darstellung nicht aufgeführt. Der besondere Vorteil dieser Art der Einbringung von Ribozym-Sequenzen in virusbefallene Pflanzen besteht darin, daß durch diese postinfektionelle Superinfektion flexibel auf die jeweilige Sequenz der zu zerstörenden viralen RNA reagiert werden kann. Dies ist besonders wichtig, da gerade RNA-Viren einer besonders hohen Mutationsrate unterliegen (Drake, 1993). Nach der Feststellung einer Virusinfektion ist man mit Hilfe der sogenannten RT-PCR in der Lage, die dominante Sequenz einer bestimmten Region der viralen RNA zu ermitteln.

Hieraus folgend kann man die Ribozymsequenz entwickeln, diese in den WL1 -Grundvektor einbringen und die zu schützenden Pflanzen durch einfaches Aufbringen der angepaßten in vitro-Transkripte vor einer sich weiter verbreitenden Virusinfektion schützen. Da die Satelliten-RNAs als Virionen in Proteine des Helfer-Virus verpackt verbreitet werden, stehen diese modifizierten ribozymkodierenden Satelliten-RNAs in neu infizierten Pflanzen bereits zur Interaktion mit der RNA des Helfer-Virus zur Verfügung.

Neben dieser Applikation der modifizierten Satelliten-RNA in Form eines in vitro-Transkripts besteht die Möglichkeit der transgenen Integration der modifizierten Satelliten-RNA in den zu schützenden Wirtspflanzenorganismus. Derlei stabile Integration und die Auswirkung der Existenz der Satelliten-RNA in einer nicht virusinfizierten Wirtspflanze wurde in der Arbeitsgruppe von Baulcombe hinreichend untersucht (Baulcombe et al., 1986).

Hierbei erfolgt die Klonierung von sogenannten "head-to-tail"-Multimeren hinter einem starken pflanzlichen Promotor (wie beispielsweise dem 35S-Promotor). Diese multimeren Transkripte mimikrieren die nach dem gegenwärtigen Modell entstehenden Replikationsintermediate und werden in Gegenwart der Helferviren korrekt zu Monomeren prozessiert. Die entstandenen Multimeren-Transkripte verursachen in den nichtinfizierten Wirtspflanzen keine Abnormalitäten hinsichtlich Wachstum, Blüten- und Samenproduktion (Baulcombe et al., 1986).

Die in den Satelliten-Molekülen integrierten Ribozym-Sequenzen sind anschließend zur Interaktion mit der korrespondierenden Sequenz des Helfervirus in der Lage. Auch die in transgenischen Pflanzen transkribierte Satelliten-RNA konnte in Virionen verpackt nachgewiesen werden (Baulcombe et al., 1986).

Der Test auf eine vorliegende Inhibierung der CMV-Replikation erfolgte durch eine Infektion mit einem CMV-Isolat in der Regel acht Wochen nach der Aussaat der Tabakpflanzen, wenn sich vier bis sechs Blätter gebildet hatten. Das unterste Blatt wurde mit einem Pinsel, der in Carborundum eingetaucht war, eingerieben. Auf diese Stelle pipettierte man 5 µg oder 25 µg Virus aus einer gereinigten Virussuspension. Bei den Kontrollpflanzen erfolgte die Behandlung mit Wasser. Die Pflanzen wurden in Klimaschränken aufbewahrt. Die Temperatur war im Tageszyklus auf 22°C und im Nachtzyklus auf 18°C eingestellt. Die Pflanzen erhielten 18 Stunden Licht und standen 8 Stunden im Dunkeln. Die Bonitur der Pflanzen ergab im Falle der durch transgen eingebrachte oder nachträglich aufgebrachte modifizierte Satelliten-RNAs eine signifikant höhere Resistenz der so behandelten virusinfizierten Pflanzen im Vergleich zu nicht behandelten virusinfizierten Kontrollpflanzen.

Isolierte Pflanzenprotoplasten (200 000/Ansatz) wurden unter Verwendung der im folgenden beschriebenen Methoden mit Satelliten-RNA und genomischen viralenRNAs transformiert (koelektroporiert).

### Protoplastenisolation

Die Protoplasten wurden aus Tabak SR1- oder Samsun-Pflanzen isoliert. Der Pflanzenkontainer, der steril gewachsene Tabakpflanzen enthielt, wurde über Nacht bei 27°C im Dunkeln gelagert. Anschließend erfolgte die Inkubation für 6-8 Stunden im Kühlschrank. Parallel erfolgte die Herstellung der benötigten 25 ml Enzymlösung, bestehend aus 150 mg Cellulysin (1560 µ) und 25 mg Macerase (80 µ). Die Lösung mußte nach vollständiger Lösung sterilfiltriert werden. 4 g Blattmaterial wurden unter sterilen Bedingungen in kleine Stücke geschnitten und in einer Petrischale mit 20 ml Präplasmolyselöung (s.u.) 20-30 Minuten ohne Lichteinfluß bei 20°C inkubiert. Anschließend wurde die Präplasmolyselösung vorsichtig abgezogen. Es erfolgte die Zugabe der Enzymlösung (s.o.) mit anschließender Inkubation ohne Lichteinfluß für 12 Stunden bei 20°C. Nach Zugabe der 20 ml Spüllösung (s.u.) wurde die verbliebene Lösung filtriert. Das Filtrat wurde auf 4 Zentrifugenröhren verteilt. Anschließend erfolgte eine Zentrifugation für 4 Minuten bei 600 rpm und einer Temperatur von 20°C. Der hiernach erhaltene Überstand wurde verworfen und das Pellet wurde in der Spüllösung resuspendiert. Die gesamte Lösung aus allen 4 Röhrchen wurde in 1 neues Zentrifugenröhrchen überführt und das Volumen wurde auf 10 ml aufgefüllt. Es folgte eine Zentrifugation für 4 Minuten bei 600 rpm und einer Temperatur von 20°C. Das resultierende Pellet wurde in 10 ml Saccharoselösung gelöst und mit 1 ml Spüllösung vorsichtig überschichtet. Es folgte eine Zentrifugation für 15 Minuten bei 400 rpm und einer Temperatur von 20°C. Die Protoplastenbande wurde vorsichtig abgezogen, die geernteten Protoplasten wurden in ein neues Röhrchen überführt und nach Zugabe von Spüllösung ad 10 ml erfolgte eine Zentrifugation für 4 Minuten bei 600 rpm und einer Temperatur von 20°C. Das Pellet wurde in 3 ml VKM⁺⁻ aufgenommen. Die erhaltenen Protoplasten wurden mit Hilfe einer Thoma-Kammer quantifiziert.

### Elektroporation

Das Volumen, das eine Menge von 200 000 Protoplasten enthielt, wurde in einem Mannit/MES-Puffer mit einem Endvolumen von 160 µl auf Eis gelagert. Die zu transformierende Nukleinsäure wurde in 160 µl KCl/Mannit Endvolumen auf Eis gelagert. Die zur Elektroporation vorgesehenen Küvetten wurden mit 70 % Ethanol gewaschen und getrocknet. Direkt vor der Elektroporation wurden die Protoplasten und die Nukleinsäuren zusammengegeben. Die Standardelektroporationsbedingungen betrugen 200 V, 25 µs (Gerätehersteller: Dialog; Gerätetyp: Elektroporator II). Die Proben wurden anschließend für 10 Minuten auf Eis und danach 5 Minuten bei 20°C inkubiert. Der Inhalt in der Küvette wurde in Eppendorfgefäße überführt und die Küvetten mit 200 µl Mannit/MES gespült. Es folgte eine Zentrifugation für 4 Minuten bei 500 rpm. Der Überstand wurde entfernt, und die Protplasten wurden erneut in 200 µl Mannit/MES aufgenommen. Die Zentrifugation wurde einmal wiederholt. Hiernach wurde eine Inkubation bei 20°C für 24 Stunden durchgeführt.

### VKM-Nutrienten-Stammlösung

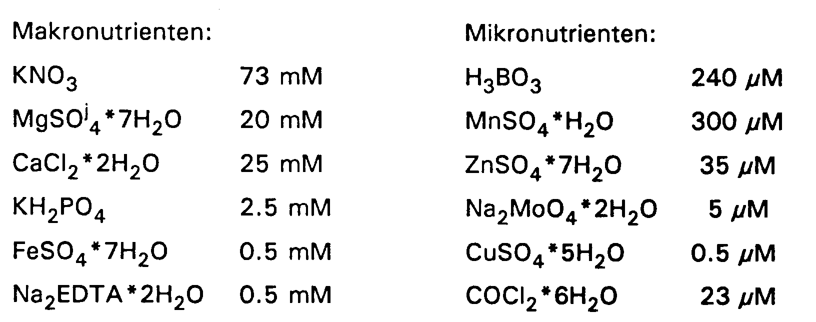

| Vitamine: | |
|---|---|
| Panthotensäure | 20 µM |
| Cholin-Chlorid | 36 µM |
| Ascorbinsäure | 57 µM |
| p-Aminobenzoesäure | 0.7 µM |
| Nicotinsäure | 40 µM |
| Vitamin B₆-HCl | 24 µM |
| Thiamin-HCl | 150 µM |
| Folsäure | 5 µM |
| Biotin | 0.2.µM |
| VitaminA-Acetat | 0.5 µM |
| Vitamin B₃ | 0.1 µM |
| Vitamin B₁₂ | 73 nM |

| VKM-Stammlösung: | |
|---|---|
| D(-)-Mannit | 7 mM |
| D(-)-Sorbit | 7 mM |
| Saccharose | 4 mM |
| D(-)-Fructose | 7 mM |
| D(-)-Ribose | 8 mM |
| D(-)-Xylose | 8 mM |
| Mannose | 7 mM |
| L( + )-Rhamnose-Monohydrat | 7 mM |
| Cellobiose | 4 mM |
| Casein-Hydrosylat | 1.25 g/l |
| myo-Inosit | 3 mM |
| Pyruvinsäure | 1 mM |
| Fumarsäure | 2 mM |
| Citronensäure | 1 mM |
| Äpfelsäure | 1.5 mM |

| VKM⁺-Kulturmedium: | |
|---|---|
| Nutrienten-Stammlösung | 100 ml/l |
| Glucose | 27 mM |
| D(-)-Mannitol | 230 mM |
| Saccharose | 30 mM |
| MES | 3 mM |
| BAP | 2.2 mM |
| Osmolarität | 460 mOsmol/kg |
| pH | 5.8 |

Das Kulturmedium wurde durch einen 0,2 µm Filter sterilfiltriert.

| Spülmedium VKM: | |
|---|---|
| Nutrienten-Stammlösung | 100 ml/l |
| KCl | 0.2 M |
| MES | 3 mM |
| Osmolarität | 460 mOsmol/kg |
| pH | 5.8 |

| Präplasmolysemedium VKM: | |
|---|---|
| Nutrienten-Stammlösung | 100 ml/l |
| Mannitol | 0.4 M |
| MES | 3 mM |
| Osmolarität | 490 mOsmol/kg |
| pH | 5.8 |

Das Kulturmedium wurde durch einen 0.2 µm Filter sterilfiltriert.

| Saccharoselösung VKM: | |
|---|---|
| Nutrienten-Stammlösung | 100 ml/l |
| Saccharose | 0.3 M |
| Osmolarität | 460 mOsmol/kg |
| pH | 5.8 |

Das Kulturmedium wurde durch einen 0.2 µm Filter sterilfiltriert.

| Enzymlösung VKM: | |
|---|---|
| Cellulysin | 6 g/l in VKM-Kulturmedium auflösen |
| Macerase | 1 g/l in VKM-Kulturmedium auflösen |

Nach Lösung der Substanzen wurde die Osmolarität sowie der pH-Wert überprüft.

Es erfolgte eine anschließende Inkubation für einen Zeitraum von 8-48 Stunden bei einer Temperatur von 25°C. Die Effekte der das Ribozym tragenden Satelliten-RNA auf die Replikation des CMV innerhalb der Protoplasten wurde mit Hilfe von gegen das Virus-Coat-Protein gerichteten Antikörpern einerseits und mit Hilfe von RNA-Isolationen mit nachfolgender Northern-Blot-Analyse andererseits quantifiziert.

### Antikörpertest

Für den Antikörpernachweis wurde ein Kit der Firma Agdia Inc. (30380 County Road 6, Elkhart, IN 46514) verwendet. Die hierin enthaltenen Antikörper sind gegen das Coat-Protein des CMV gerichtet. Für den jeweiligen Antikörpertest wurde die jeweils pro Elektroporation verwendete gesamte Protoplastenpopulation eingesetzt.

### Northern-Blots

Die gesamte zur Elektroporation eingesetzte Protoplastenpopulation wurde zur RNA-Extraktion eingesetzt. Die Protoplasten wurden mit dem gleichen Volumen eines Aufschlußpuffers (200 mM NaCl, 100 mM Tris-HCl, pH 7.5, 1 mM EDTA) gemischt und mit einer Phenol/Chloroform-Lösung nach der Einstellung der wäßrigen Phase auf eine 1 %igen SDS-Endkonzentration kurz geschüttelt und anschließend in einer Eppendorf-Zentrifuge für 5 Minuten zentrifugiert. Die wäßrige Phase wurde mit einem gleichen Volumen Chloroform versetzt, geschüttelt und erneut für 5 Minuten zentrifugiert. Die wäßrige Phase wurde mit 1/10 Volumen einer 3 M Natrium-Acetat-Lösung, pH 4.8 versetzt und mit 3 Volumen 96 %igem Ethanol, p.a. versetzt. Nach einer Fällungsdauer von 1 Stunde bei -80°C erfolgte eine Zentrifugation für 15 Minuten. Anschließend wurde das Pellet in einer 70 %igen Ethanollösung gewaschen, getrocknet und anschließend in einem Volumen von 100 µl TE, pH 7.5 gelöst. Für den Fall, daß noch zuviel DNA in dieser Lösung enthalten sein sollte, wurde eine DNase-Behandlung (RNase frei DNase) durchgeführt. Nach einer Phenol/Chloroform-Extraktion (s.o.) wurde die Nukleinsäure auf einem Agarose/Formaldehyd-Gel pherographiert. Es erfolgte ein Transfer auf eine Nylonmembran und eine Hybridisierung mit radioaktiv markierten Transkripten, die eine zur genomischen Virus-RNA komplementäre Sequenz aufweisen.

Durch die Entnahme von Protoplasten zu verschiedenen Zeitpunkten konnte neben der Quantifizierung auch der zeitliche Verlauf der Inhibition verfolgt werden.

Neben dieser hier am Beispiel der CARNA5 Satelliten-RNA und des korrespondierenden CMV-Helfervirus aufgezeigten Inhibierung der Virusreplikation sind natürlich auch andere Helfervirus/Satelliten-RNA-Kombinationen denkbar, die ebenfalls den Schutz vor einer systemischen Infektion und damit den Schutz vor einer weiteren Verbreitung eines bestimmten Virus ermöglichen.

### 2.2 Anwendung der Technolgie mit Satelliten-Viren

Im Gegensatz zu den Satelliten-RNAs, die für keine eigenen Genprodukte kodieren, weisen die Satelliten-Viren ein offenes Leseraster auf, das am 5'-seitigen Ende der genomischen RNA lokalisiert ist. Dieses offene Leseraster kodiert für ein Hüllprotein dieser Satelliten-Viren. Somit sind diese Satelliten-Viren zwar auf die Anwesenheit sogenannter Helfer-Viren angewiesen, werden jedoch nicht in deren "Capsid" verpackt. Das 3'-seitige Ende der genomischen RNA kodiert kein offenes Leseraster, und es steht somit für die Einbringung zusätzlicher RNA-Sequenzen zur Verfügung.

Satelliten-Viren sind auf Grund ihrer Kodierungskapazität für ein Protein auch um einiges größer als die Satelliten-RNAs. Für den Satelliten des "Tobacco Necrosis Virus", das STNV, konnte gezeigt werden, daß kleinere Insertionen in der nicht kodierenden 3'-Region der RNA des Satelliten-Virus in den meisten Fällen keinen Einfluß auf die Produktion der RNA des Satelliten-Virus im Rahmen der Replikation hatten. Derlei integrierte Sequenzen werden dann repliziert und als fester Bestandteil in der Nachkommenschall stabil vererbt.

Im Gegensatz zur möglichen Insertion von kurzen Sequenzen in die nicht kodierende Region sollte eine Insertion in den kodierenden 5'-Bereich vermieden werden, da hierdurch das offene Leseraster zerstört wird (Van Emmelo et al., 1987). Diese Veränderung beeinflußt zwar nicht das Auttreten der Replikationsintermediate, wohl aber die Verbreitung der Satelliten-Viren, da das zur Verpackung notwendige Hüllprotein nicht ordnungsgemäß gebildet werden kann.

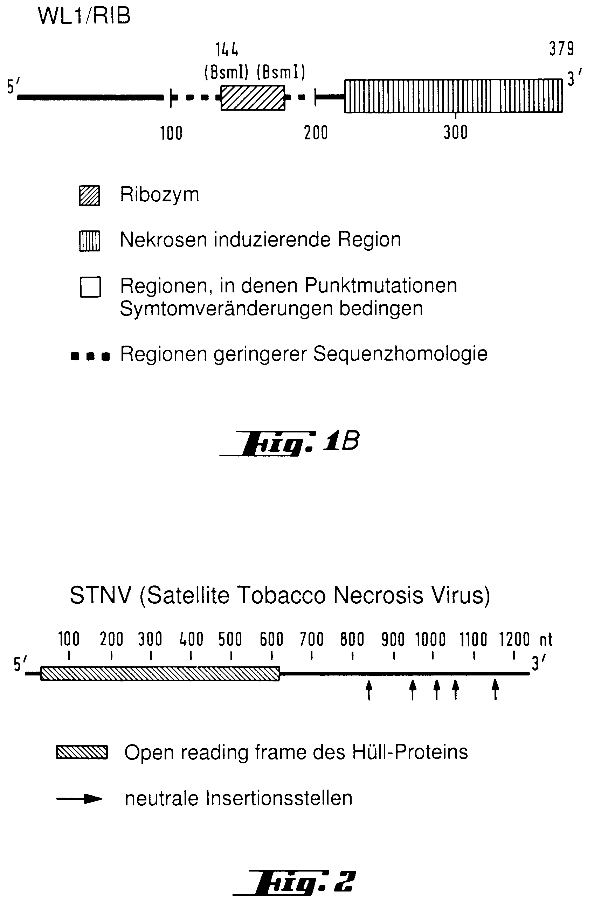

In Abbildung 2 ist der Aufbau eines Satelliten-Virus grob schematisch am Beispiel des STNV gezeigt. Der verstärkt balkenförmig dargestellte Bereich entspricht dem "ORF" des Hüllproteins.

Die gefundenen neutralen Insertionsstellen sind durch die Positionierung von Pfeilen gekennzeichnet (van Emmelo et al., 1987). An diesen Positionen sollte somit die Insertion eines Ribozyms möglich sein, das dann ebenfalls stabil auf die Nachkommenschaft vererbt wird und durch die Verpackung in Virionen des Satelliten-Virus auch systemische Verbreitung findet.

Mit der eine Ribozym-Sequenz enthaltenden cDNA der Satelliten-Viren können mit dem Helfer-Virus infizierte Pflanzen entweder direkt inokuliert werden, oder aber es werden von der klonierten cDNA in vitro-Transkripte hergestellt, die dann für eine Inokulation eingesetzt werden können.

Neben der postinfektionellen Superinfektion ist auch im Falle der Klonierung einer Ribozym-Sequenz in die cDNA eines Satelliten-Virus eine genomische Integration einer derartig modifizierten cDNA möglich, wodurch eine permanente Transkription der modifzierten RNA des Satelliten-Virus eine erhöhte Resistenz gegen das Helfer-Virus zur Folge hat.

Die Klonierung erfolgt mit Hilfe eines binären Pflanzenvektors, der über einen starken Promotor wie z.B. den 35S-Promotor des CaMV sowie die entsprechenden Terminationssequenzen verfügt.

Neben dem hier beschriebenen Satelliten Virus STNV des Tobacco Necrosis Virus (TNV) können außerdem die folgenden Viren vorzugsweise zur Herstellung der erfindungsgemäßen Ribozyme verwendet werden: das Satelliten Virus des Maize White Line Mosaic Virus (MWLMV), das SV-MWLMV (Zhang et al., 1991) bzw. das Satelliten-Virus des Panicum Mosaic Virus (PMV), das SPMV (Masuta et al., 1987).

Auch diese Satelliten-Viren eignen sich für den Einbau von Ribozym-Sequenzen in nicht kodogene Bereiche der RNA, wenn sie sich auch in ihrer Genomstruktur und Größe alle etwas voneinander unterscheiden.

### 2.3 Ausschaltung endogen kodierter Genprodukte

Im Gegensatz zur Anwendung der Satelliten-RNAs und Satelliten-Viren als Koinfektion eines Helfer-Virus ist der Einsatz modifizierter, ribozymenthaltender Satelliten-RNAs und Satelliten-Viren ebenfalls in Form einer spezifischen Ausschaltung endogen kodierter RNAs denkbar. Hierzu werden die für die Replikation und die Verpackung notwendigen viralen RNAs als cDNA im Genom des Wirtsorganismus stabil integriert, und durch die Vorschaltung eines starken Promotors wird die starke Transkription der für die essentiellen viralen Proteine kodierenden RNA gewährleistet. Diesen DNA-Sequenzen fehlen jedoch die 5'- und 3'-terminalen Sequenzbereiche, da andererseits sonst auch diese viralen RNAs repliziert und in Virionen verpackt werden könnten, was zu einer systemischen Infektion des Wirtsorganismus und zu einer Übertragung auf andere Organismen führen würde. Die Satelliten-RNAs wie auch die Satelliten-Viren hingegen könnten systemisch verbreitet werden, und somit zur Ausschaltung spezifischer Genprodukte in dem vollständigen jeweiligen Organismus führen. Der Vorteil gegenüber einfachen transgenen Organismen, die für ein Ribozym kodieren, besteht darin, daß die einmalige Grundausstattung mit den notwendigen Genen des jeweiligen Helfer-Virus immer identisch ist, und man somit diese Konstruktion nur einmal vornehmen muß. Angepaßt an die jeweiligen Absichten oder Erfordernisse werden anschließend modifizierte Satelliten-RNAs oder RNA-Moleküle von Satelliten-Viren in den Organismus eingebracht, die dann durch die funktionellen Moleküle des Helfer-Virus am Wirkungsort stark amplifiziert werden.

Als Klonierungsvehikel für diese Anwendung kommen im Falle einer Anwendung im pflanzlichen tierischen und humanen Bereich alle Satelliten-Viren in Betracht, wobei im pflanzlichen Anwendungsbereich zusätzlich modifizierte Satelliten-RNAs für diese Zielsetzung verwendet werden können.

### 3. Legende zu den Abbildungen

- Abbildung 1:: Vergleich verschiedener Satelliten-RNA-Subspezies
- 1 A:: Y-satRNA (groß, nekrotisch, chlorotisch) B5-satRNA (klein, chlorotisch) D/WL1/I17N-satRNAs (klein, nekrotisch)
- 1B:: Beispiel des CARNA5
- Abbildung 2:: Aufbau eines Satelliten-Virus grob schematisch am Beispiel des STNV gezeigt. Der verstärkt balkenförmig dargestellte Bereich entspricht dem "ORF" des Hüllproteins.

### 4. Referenzen

Baulcombe, D.C., Saunders, G.R., Bevan, M.W., Mayo, M.A., Harrison, B.D. (1986), Nature, 321, 446 - 449
Bems, K.I., (1990), Microbiological Reviews, 54, 316 - 329
Collmer, C.W., Howell, S.H. (1992), Ann. Rev. Phytopathol. 30, 419 - 442
Drake, J.W. (1993), Proc. Natl. Acad. Sci. USA, 90, 4171 - 4178
Edgington, S.M. (1992), Biotechnology, 10, 256 - 262
Kaper, J.M., Collmer, C.W. (1988), In: RNA genetics, Vol. III, 317 - 343
Eds: Domingo, J., Holland, J., Ahlquist, P., CRC Press, Boca Raton
Kaper, J.M., Tousignant, M.E., Steen, M.T. (1988), Virology, 163, 284 - 292
Kaper J.M., Tousignant, M.E. Geletka, L.M. (1990), Res. Virol., 14, 487 - 503
Montasser, M.S., tousignant, M.E., Kaper, J.M. (1991), Plant Dis. 75, 86 - 92
Palukatitis, P. (1988), Mol. Plant-Microbe Interact. 1, 175 - 181
Roossinck, M.J., Sleat, D., Palukaitis, P. (1992), Microbiological Reviews, 56, 256-279
Sleat. D.E., Palukaitis, P. (1990), Proc. Natl. Acad. Sci. USA, 87, 2946-2950
Symons, R.H. (1991), Critical Reviews in Plant Sciences, 10, 189-234
Taylor, J. (1990), Sem. Virol., 1, 135-141
Tien. P., Zhang, X., Oui, B., Qin, B., Wu, G. (1987), Ann. Appl. Biol., 111, 143-152
Van Emmelo, J., Ameloot, P., Fiers, W. (1987), Virology, 157, 480-487
Zhang, L., Zitter, T.A., Palukaitis, P. (1991), Virology, 180, 467-473

## Patentansprüche

1. Modifizierte Ribozyme, enthaltend
(a) nukleolytisch aktive RNA-Sequenzen, die virale genomische RNA, mRNA oder hnRNA spalten und
(b) Satelliten-Viren oder Satelliten-RNA, die mit den nukleolytisch aktiven RNA-Sequenzen funktionell verknüpft sind.

2. Verfahren zur Herstellung modifizierter Ribozyme, dadurch gekennzeichnet, daß
(a) nukleolytisch aktive RNA-Sequenzen, die virale genomische RNA, mRNA oder hnRNA spalten können
(b) mit Satelliten-Viren oder Satelliten-RNA funktionell verknüpft werden.

3. Verfahren gemäß Anspruch 2, in denen die nukleolytisch aktiven RNA-Sequenzen in Satelliten-RNAs sowie in die RNA von Satelliten-Viren pflanzenpathogener, tierpathogener und humanpathogener Viren integriert werden.

4. Verfahren gemäß Anspruch 2, dadurch gekennzeichnet, daß nukleolytisch aktiven RNA-Sequenzen verwendet werden, die Hammerhead-oder Hairpin-Motive aufweisen, RNAseP-ähnliche Sequenzen oder Hepatitis-Delta-Virus-Sequenzmotive aufweisen oder den Ribozymen vom Typ der Gruppe-I-Introns zugeordnet werden können.

5. Verfahren zur Herstellung virusresistenter Pflanzen, dadurch gekennzeichnet, daß
(a) Pflanzenzellen mit der für die enzymatischen RNA-Moleküle kodierenden DNA transformiert werden,
(b) die DNA stabil in das Genom der Pflanzen integiert wird und
(c) aus diesen Zellen Pflanzen regeneriert werden.

6. Verfahren zur Behandlung von Pflanzen, dadurch gekennzeichnet, daß Pflanzen mit den modifizierten Ribozymen äußerlich behandelt werden.

7. DNA-Fragmente, die für die modifizierten Ribozyme gemäß Anspruch 1 kodieren.

8. Verwendung der modifizierten Ribozyme als antivirale Arzneimittel.

9. Verwendung der modifizierten Ribozyme als antivirale Mittel gegen pflanzenpathogene Viren .
